# FASCICULE DE BREVET EUROPEEN

(11) **EP 1 948 092 B1**
(45) Date de publication et mention de la délivrance du brevet: **17.08.2016**
(21) Numéro de dépôt: 06820882.6
(22) Date de dépôt: 14.11.2006
(51) Int. Cl.: A61F 2/42

(54) **IMPLANT, NOTAMMENT IMPLANT PARTIEL DE LA TÊTE DU CUBITUS**
IMPLANTAT, INSBESONDERE TEILIMPLANTAT FÜR DEN ULNAKOPF
IMPLANT, ESPECIALLY PARTIAL IMPLANT OF THE HEAD OF THE ULNA

(30) Priorité: 17.11.2005 FR 0511671
(43) Date de publication de la demande: 30.07.2008
(73) Titulaire: TORNIER, 38330 Montbonnot-Saint-Martin (FR)
(72) Inventeur: HASSLER, Michel, F-38330 Saint-Ismier (FR); REAL, Cécile, 75018 Paris (FR); GARCIA-ELIAS, Marc, E-08230 Matadepera (ES)
(74) Mandataire: Lavoix
(86) Numéro de dépôt international: PCT/IB2006/003190
(87) Numéro de publication internationale: WO 2007/057741

(56) Documents cités:
- EP-A1- 0 454 646
- EP-A1- 0 749 735
- WO-A-01/70138
- WO-A-91/16017
- DE-A1- 19 620 525
- FR-A1- 2 800 269
- US-A- 5 735 905
- US-A- 5 938 699
- US-B1- 6 302 915
- K.D. GORDON ET AL.: "Kinematics of Ulnar Head Arthroplasty" JOURNAL OF HAND SURGERY, vol. 28b, no. 6, 6 décembre 2003 (2003-12-06), pages 551-558, XP002435195 cité dans la demande

## Description

La présente invention concerne un implant, notamment un implant partiel de la tête du cubitus.

Un implant partiel de la tête du cubitus est décrit dans l'article intitulé « Kinematics of ulnar head arthroplasty » de K.D. Gordon et al. publié dans « The journal of hand surgery », vol. 28B No. 6, décembre 2003. Il comprend une tête hémisphérique destinée à remplacer la partie articulaire de la tête du cubitus, c'est-à-dire la partie latérale de l'extrémité distale du cubitus articulée à la tête du radius. La tête hémisphérique est emmanchée sur l'extrémité d'une tige et rigidement fixée à celle-ci au moyen d'une vis, la tige étant quant à elle destinée à être fixée rigidement à l'intérieur d'un trou formé dans le cubitus pour maintenir l'implant en place.

A la différence d'un implant total de la tête du cubitus, un tel implant partiel permet de préserver le ligament triangulaire, qui relie la tête du cubitus et le radius, le ligament ulnocarpien, qui relie le cubitus et le carpe, et de maintenir tendue la membrane interosseuse reliant le cubitus et le radius. La stabilité d'un tel implant partiel est donc bien supérieure à celle d'un implant total.

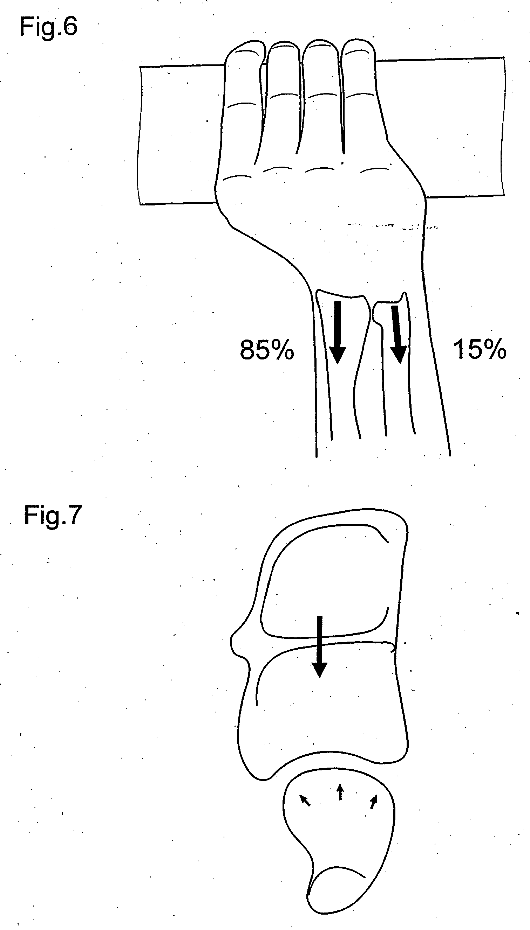

On sait que la tête du cubitus est davantage soumise à des charges transversales qu'à des charges axiales. Il a en effet été observé qu'environ 15% seulement des charges axiales appliquées à l'extrémité distale de l'avant-bras sont reçues par le cubitus, les 85 autres pourcents étant reçus par le radius. Les charges transversales sont particulièrement élevées lors de rotations de l'avant-bras (pronation, supination). Aux figures 6 et 7 on a représenté, à titre d'illustration, les charges axiales reçues par le cubitus et le radius dans une position verticale de la main (figure 6) et les charges transversales que s'appliquent mutuellement la tête du cubitus et la tête du radius dans une position horizontale de l'avant-bras (figure 7).

Dans le cas de l'implant partiel évoqué ci-dessus, les charges transversales reçues par la tête de l'implant sont transmises â la tige. Ceci a pour conséquence que la partie restante de la tête du cubitus, de même que les ligaments environnants, ne reçoivent plus les charges qu'ils recevaient initialement. N'étant plus sous pression, la partie restante de la tête du cubitus est condamnée à se décalcifier progressivement et les ligaments environnants à perdre de leur vigueur.

WO-A-91/16017, sur lequel est basé le préambule de la revendication 1 annexée, divulgue un implant total de la tête du cubitus, comprenant une tête, liée fixement au radius et connectée à une tige qui est fixée rigidement au cubitus dont la tête a été préalablement réséquée en totalité. La tête de cet implant est conçue mobile en translation par rapport à la tige dans une direction transversale aux os du radius et du cubitus, autrement dit dans une direction parallèle aux charges transversales susmentionnées. Cette mobilité translative interne de l'implant autorise le radius de pouvoir tourner autour du cubitus lors d'une rotation du poignet du patient. Toutefois cette solution ne remédie pas à l'inconvénient présenté plus haut, relatif à la dévitalisation de la région de l'extrémité du cubitus.

La présente invention vise à remédier à cet inconvénient ou à tout le moins à le réduire.

A cette fin il est proposé un implant, notamment un implant partiel de la tête du cubitus, tel que défini à la revendication 1.

Grâce à cette mobilité en translation de la tête de l'implant, cette dernière peut venir s'appuyer contre la partie restante de l'os sur lequel elle est montée et/ou contre les ligaments environnants lors de mouvements du patient et peut ainsi leur transmettre les charges transversales qu'elle reçoit. De préférence, pour éviter que la tête de l'implant ne s'use lors de ses déplacements par rapport à l'élément de maintien ou n'use les os avoisinants, ladite tête est réalisée en pyrocarbone et sa surface extérieure est polie.

L'élément de maintien est avantageusement une tige dont l'axe est transversal à la direction précitée et qui est conçue pour être maintenue simplement par déformation élastique dans un trou préalablement formé dans ledit os. Cette tige est par exemple solidaire d'un téton engagé dans un alésage de la tête, la mobilité en translation transversale de la tête par rapport à la tige étant obtenue par un jeu entre le téton et la tête. De préférence, les sections respectives du téton et de l'alésage sont toutes deux circulaires et la section du téton a un diamètre inférieur à celui de la section de l'alésage.

D'autres caractéristiques et avantages de la présente invention apparaîtront à la lecture de la description détaillée suivante faite en référence aux dessins schématiques annexés dans lesquels :
- la figure 1 montre en perspective le radius, le cubitus et un implant partiel selon l'invention remplaçant la partie articulaire de la tête du cubitus ;
- la figure 2 montre en vue frontale l'extrémité distale du radius et du cubitus ainsi que l'implant partiel selon l'invention remplaçant la partie articulaire de la tête du cubitus ;
- la figure 3 est une vue en coupe de l'implant partiel selon l'invention selon l'axe III-III de la figure 5 ;
- la figure 4 est une vue plane, partiellement coupée, de l'implant partiel selon l'invention suivant la flèche IV de la figure 5 ;
- la figure 5 montre l'implant partiel selon l'invention en vue de dessus ;
- la figure 6, déjà commentée, est une vue montrant la répartition des charges axiales reçues par l'extrémité distale du radius et du cubitus dans une position verticale de la main ; et
- la figure 7, déjà commentée, est une vue en coupe transversale montrant les charges transversales que s'appliquent mutuellement la tête du radius et la tête du cubitus dans une position de pronosupination neutre avec l'avant-bras horizontal.

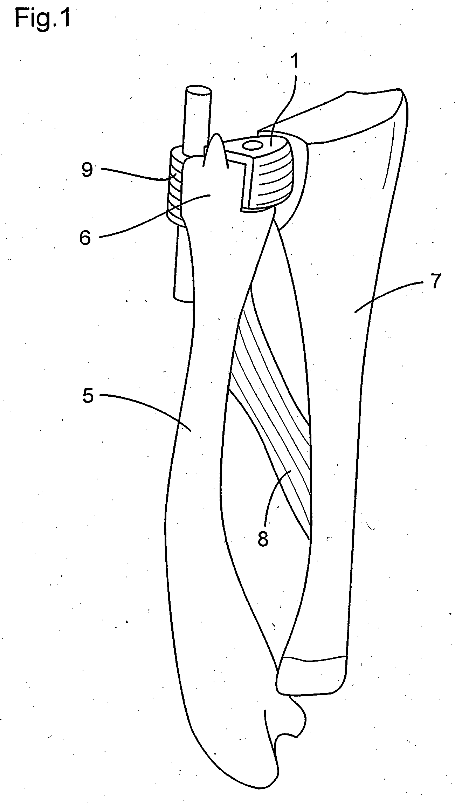

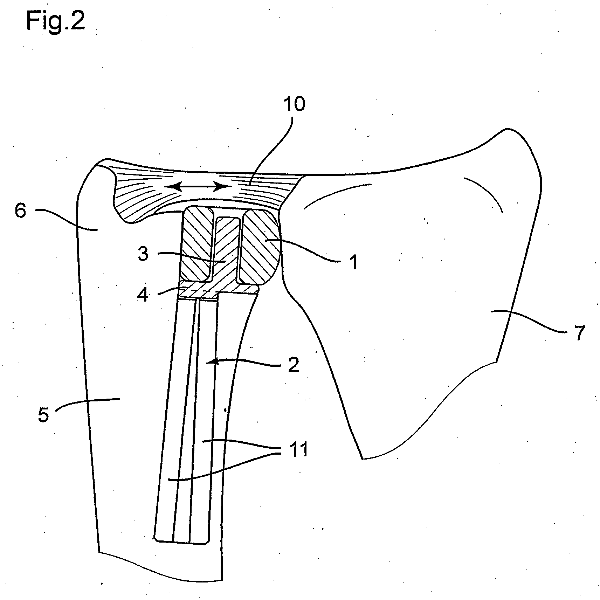

En référence aux figures 1 à 5, un implant partiel de la tête du cubitus selon l'invention comprend une tête 1 connectée à une tige 2 au moyen d'un téton 3 faisant saillie sur un plateau 4 situé à une extrémité de la tige 2. L'axe du téton 3 est parallèle, mais non confondu, avec l'axe de la tige 2. La tête 1 est destinée à être placée dans un espace obtenu en retirant la partie articulaire de la tête du cubitus, comme montré aux figures 1 et 2 dans lesquelles le repère 5 désigne le cubitus, le repère 6 la partie restante de la tête du cubitus, le repère 7 le radius, le repère 8 la membrane interosseuse, le repère 9 la gaine du tendon cubital postérieur et le repère 10 le ligament triangulaire. La tige 2, quant à elle, est destinée à être introduite dans un trou correspondant formé préalablement dans le cubitus pour maintenir l'implant en place.

La tige 2, le téton 3 et le plateau 4 forment de préférence une seule pièce, réalisée en métal, par exemple en titane. A la différence des implants conventionnels, l'implant selon l'invention n'a pas besoin d'être solidement fixé à l'os sur lequel il est monté, en l'occurrence le cubitus. La tige 2 n'a pas pour but de résister à des contraintes élevées mais simplement de positionner l'implant afin d'empêcher sa luxation pendant des mouvements de pronation et supination. Cette tige 2 peut ainsi être constituée de bras élastiques 11, au nombre de quatre dans l'exemple illustré, se déformant lors de leur insertion dans le cubitus pour maintenir l'implant en place uniquement par leur déformation élastique. On notera que la stabilité de l'implant selon l'invention est assurée avant tout par la partie restante 6 de la tête du cubitus et par les ligaments environnants. En particulier, le ligament triangulaire 10 retient l'implant dans la direction axiale (cf. figure 2).

De préférence, la tête 1 de l'implant selon l'invention est réalisée en pyrocarbone et sa surface extérieure est polie. On sait que le pyrocarbone a un module d'élasticité (module de Young) voisin de celui de l'os, ce qui lui permet, surtout si sa surface est polie, de frotter contre l'os sans engendrer d'usure ni de l'os ni de lui-même. Selon un mode de réalisation typique, la tête 1 est constituée d'un noyau en graphite recouvert d'une couche de pyrocarbone, l'ensemble graphite - pyrocarbone ayant un module d'élasticité (module de Young) voisin de celui de l'os. Selon un autre mode de réalisation, la tête 1 est en pyrocarbone massif, c'est-à-dire est dépourvue de noyau en graphite.

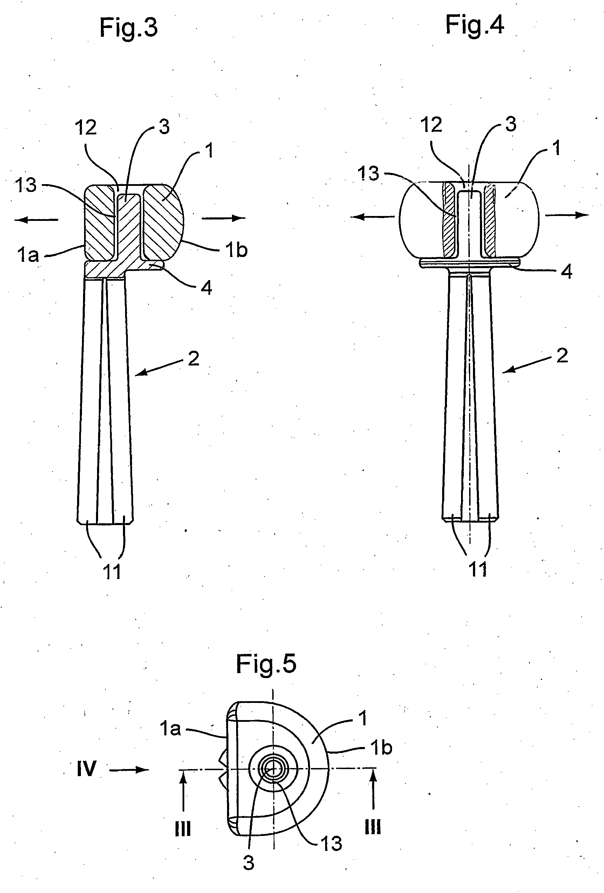

La tête 1 a sensiblement la forme d'une hémisphère tronquée par deux plans parallèles entre eux et perpendiculaires à la face plane de l'hémisphère et à l'axe de la tige 2. La face plane 1a et la face hémisphérique 1b de la périphérie de la tête 1 sont destinées à être placées en regard, respectivement, de la partie restante 6 de la tête du cubitus et de la tête du radius 7. On notera que la tête 1 n'a pas besoin d'avoir une forme respectant précisément l'anatomie de la tête du cubitus, le rôle de cette tête 1 étant, comme on le verra ci-dessous, de servir de pièce d'interposition entre la tête du radius et la partie restante 6 de la tête du cubitus. Un alésage 12 est formé dans la tête 1 pour recevoir le téton 3 et connecter ainsi la tête 1 à la tige 2. Conformément à l'invention, cet alésage 12 a une plus grande section que le téton 3 de façon à créer un jeu 13 entre la tête 1 et le téton 3 rendant la tête 1 mobile transversalement par rapport à l'axe de la tige 2, comme montré par les flèches sur les figures 3 et 4. Dans le cadre de la présente invention, la notion de mobilité transversale s'entend d'une mobilité en translation dans un plan coupant l'axe de la tige 2 et parallèle aux charges transversales appliquées par la tête du radius lorsque l'implant est en place dans le patient. Dans l'exemple illustré, où la tige 2 et le téton 3 sont parallèles, le plan précité est sensiblement perpendiculaire aux axes respectifs de la tige 2 et du cubitus.

Dans un mode de réalisation typique de la présente invention, le jeu 13 entre la tête 1 et le téton 3 est d'environ 1 mm. De préférence, les sections respectives de l'alésage 12 et du téton 3 sont toutes deux circulaires, mais de diamètres différents, permettant ainsi à la tête 1 de se déplacer dans toutes les directions d'un plan transversal à la tige 2. En variante toutefois, la section de l'alésage 12 pourrait avoir une autre forme, par exemple une forme oblongue, pour privilégier une direction particulière de déplacement transversal de la tête 1.

La fonction du téton 3 est simplement de limiter les déplacements transversaux de la tête 1. On notera que la tête 1 n'a pas besoin d'être rendue solidaire axialement de la tige 2. Une fois l'implant mis en place, en effet, la tête 1 est retenue axialement par le ligament triangulaire 10. Pour les cas où le ligament triangulaire 10 est endommagé, on pourrait néanmoins prévoir une variante dans laquelle la tête 1 serait bloquée axialement par rapport à la tige 2, par exemple par un renflement prévu sur le pourtour du téton 3 ou par une plaque fixée à l'extrémité du téton 3 opposée au plateau 4.

Comme indiqué précédemment, la tête 1 de l'implant selon l'invention est destinée à s'interposer entre la partie articulaire de la tête du radius 7 et la partie restante 6 de la tête du cubitus 5, la surface hémisphérique 1b de la tête 1 servant de surface d'articulation avec la tête du radius 7. Du fait que la partie 6 de la tête du cubitus 5 est conservée, le ligament triangulaire 10 qui relie le cubitus et le radius ainsi que le ligament ulnocarpien (non visible sur les dessins) et la gaine du tendon cubital postérieur 9 sont préservés et la membrane interosseuse 8 reste tendue. Tous ces ligaments 8-10 peuvent ainsi continuer à remplir leur fonction.

Il est généralement constaté que lorsqu'une affection touche la tête du cubitus, le ligament triangulaire 10 est distendu. On choisit donc, pour la tête 1 de l'implant, une taille dans le plan transversal légèrement supérieure à la taille de la partie de tête du cubitus qu'elle remplace. Ainsi, la présence de la tête 1 maintient les têtes du cubitus et du radius légèrement écartées l'une de l'autre par rapport à leur position anatomique, comme montré par la double flèche sur la figure 2, ce qui retend le ligament triangulaire 10.

La mobilité transversale de la tête 1 par rapport à la tige de maintien 2 présente plusieurs avantages. En premier lieu, elle réduit les charges que reçoit la tige 2 lorsque la tête du cubitus est soumise à des contraintes transversales, en particulier lors de mouvements de rotation de l'avant-bras (pronation, supination), et augmente celles que reçoit la partie restante 6 de la tête du cubitus et les ligaments environnants. En effet, au lieu de transmettre l'essentiel des contraintes transversales reçues à la tige 2, la tête 1, en se déplaçant transversalement, vient s'appuyer contre la partie restante 6 de la tête du cubitus et/ou contre les ligaments environnants, lesquels reçoivent ainsi des contraintes proches de celles qu'ils recevaient avant le retrait de la partie articulaire de la tête du cubitus. La stabilité d'origine de la tête du cubitus est donc respectée. La partie restante 6 de la tête du cubitus, du fait qu'elle continue à subir des contraintes, conserve sa substance. Quant aux ligaments environnants, mis en tension par les mouvements transversaux de la tête 1, ils restent eux aussi actifs.

Un autre avantage de la mobilité transversale de la tête 1, plus précisément du jeu entre la tête 1 et le téton 3, est qu'elle permet au chirurgien, une fois la tige 2 introduite dans le cubitus, de placer facilement une tête 1 sur la tige 2 puis de la retirer tout aussi facilement si besoin est. Le chirurgien peut ainsi essayer plusieurs tailles de tête jusqu'à trouver celle qui convient le mieux au patient.

Bien qu'elle ait été décrite ci-dessus dans le cadre d'un implant partiel de la tête du cubitus, il est clair que la présente invention peut s'appliquer de la même manière à tout implant, partiel ou total, dont la tête est destinée à remplacer au moins une partie articulaire latérale d'un os et à recevoir des charges transversales.

## Revendications

1. Implant comprenant une tête (1) destinée à remplacer au moins une partie articulaire latérale d'un os recevant des charges transversales et un élément de maintien (2) connecté à la tête (1) et destiné à être fixé audit os, la tête (1) étant mobile en translation par rapport à l'élément de maintien (2) dans au moins une direction qui, lorsque l'implant est en place dans le patient, est parallèle auxdites charges transversales,
**caractérisé en ce que** la tête (1) est pourvue, sur sa périphérie extérieure, de deux faces opposées (1 a, 1 b), à savoir une face d'articulation, contre laquelle lesdites charges transversales sont appliquées dans ladite direction, et une face d'appui contre une partie restante de l'os et/ou contre des ligaments environnants.

2. Implant selon la revendication 1, **caractérisé en ce que** la tête (1) est en pyrocarbone.

3. Implant selon la revendication 2, **caractérisé en ce que** la surface extérieure de la tête (1) est polie.

4. Implant selon l'une quelconque des revendications 1 à 3, **caractérisé en ce que** l'élément de maintien (2) est une tige.

5. Implant selon la revendication 4, **caractérisé en ce que** ladite direction dans laquelle la tête (1) est mobile en translation est transversale par rapport à l'axe de la tige (2).

6. Implant selon la revendication 5, **caractérisé en ce que** la tige (2) est solidaire d'un téton (3) engagé dans un alésage (12) de la tête (1), la mobilité en translation transversale de la tête (1) par rapport à la tige (2) étant obtenue par un jeu (13) entre le téton (3) et la tête (1).

7. Implant selon la revendication 6, **caractérisé en ce que** les sections respectives du téton (3) et de l'alésage (12) sont toutes deux circulaires et la section du téton (3) a un diamètre inférieur à celui de la section de l'alésage (12).

8. Implant selon l'une quelconque des revendications 4 à 7, **caractérisé en ce que** la tige (2) est conçue pour être maintenue dans un trou dudit os simplement par déformation élastique.

9. Implant selon l'une quelconque des revendications 1 à 8, **caractérisé en ce qu'**il consiste en un implant partiel.

10. Implant selon l'une quelconque des revendications 1 à 8, **caractérisé en ce qu'**il consiste en un implant de la tête du cubitus, et **en ce que** la face d'articulation (1 b) de la tête (1) est prévue pour s'articuler avec la tête du radius (7).

11. Implant selon la revendication 10, **caractérisé en ce qu'**il consiste en un implant partiel de la tête du cubitus, et **en ce que** la face d'appui (1 a) est prévue pour s'appuyer contre une partie restante (6) de la tête du cubitus (5).

12. Implant selon l'une quelconque des revendications précédentes, **caractérisé en ce que** la face d'articulation (1 b) de la tête (1) est hémisphérique, et **en ce que** la face d'appui (1 a) est plane.

## Patentansprüche

1. Implantat, umfassend einen Kopf (1) zum Ersetzen mindestens eines seitlichen Gelenkteils eines querbelasteten Knochens und ein Halteelement (2), das mit dem Kopf (1) verbunden ist und zur Befestigung an dem Knochen bestimmt ist, wobei der Kopf (1) bezogen auf das Halteelement (2) in mindestens eine Richtung, welche parallel zu der Querbelastung verläuft, translatorisch beweglich ist, wobei das Implantat im Patienten angebracht ist,
**dadurch gekennzeichnet, dass** der Kopf (1) an seinem Außenumfang mit zwei gegenüberliegenden Seiten (1a, 1b) versehen ist, nämlich einer Gelenkseite, gegen welche die Querbelastung in vorgenannter Richtung wirkt, und einer Stützseite zur Anlage gegen den übrigen Teil des Knochens und/oder gegen die umliegenden Bänder.

2. Implantat nach Anspruch 1, **dadurch gekennzeichnet, dass** der Kopf (1) aus Pyrokohlenstoff ist.

3. Implantat nach Anspruch 2, **dadurch gekennzeichnet, dass** die Außenfläche des Kopfes (1) poliert ist.

4. Implantat nach einem der Ansprüche 1 bis 3, **dadurch gekennzeichnet, dass** das Halteelement (2) eine Stange ist.

5. Implantat nach Anspruch 4, **dadurch gekennzeichnet, dass** die Richtung, in welche der Kopf (1) translatorisch bewegbar ist, bezogen auf die Achse der Stange (2) quer verläuft.

6. Implantat nach Anspruch 5, **dadurch gekennzeichnet, dass** die Stange (2) fest mit einem in einer Bohrung (12) des Kopfes (1) angebrachten Bolzen (3) verbunden ist, wobei die translatorische Querbeweglichkeit des Kopfes (1) bezogen auf die Stange (2) durch ein Spiel (13) zwischen dem Bolzen (3) und dem Kopf (1) erreichbar ist.

7. Implantat nach Anspruch 6, **dadurch gekennzeichnet, dass** die jeweiligen Abschnitte des Bolzens (3) und der Bohrung (12) beide kreisförmig sind und der Abschnitt des Bolzens (3) einen kleineren Durchmesser als der Abschnitt der Bohrung (12) aufweist.

8. Implantat nach einem der Ansprüche 4 bis 7, **dadurch gekennzeichnet, dass** die Stange (2) derart konzipiert ist, dass sie in einem Loch des Knochens lediglich durch elastische Verformung haltbar ist.

9. Implantat nach einem der Ansprüche 1 bis 8, **dadurch gekennzeichnet, dass** das Implantat aus einem Teilimplantat besteht.

10. Implantat nach einem der Ansprüche 1 bis 8, **dadurch gekennzeichnet, dass** das Implantat aus einem Implantat des Ulnakopfes besteht und dass die Gelenkseite (1b) des Kopfes (1) für eine gelenkige Verbindung mit dem Speichenkopf (7) vorgesehen ist.

11. Implantat nach Anspruch 10, **dadurch gekennzeichnet, dass** das Implantat aus einem Teilimplantat des Ulnakopfes besteht und dass die Stützseite (1a) für die Abstützung gegen den übrigen Teil (6) des Ulnakopfes (5) vorgesehen ist.

12. Implantat nach einem der vorangehenden Ansprüche, **dadurch gekennzeichnet, dass** die Gelenkseite (1b) des Kopfes (1) eine Halbkugel ist und dass die Stützseite (1a) eben ist.

## Claims

1. Implant comprising a head (1) intended to replace at least a lateral articular portion of a bone subject to transverse loads, and a holding element (2) connected to the head (1) and intended to be fixed to said bone, the head (1) being movable in terms of translation relative to the holding element (2) in at least one direction which, when the implant is in place in the patient, is parallel to said transverse loads,
**characterised in that** the head (1) is provided, on its external periphery, with two opposite faces (1a, 1b), namely an articulation face, against which said transverse loads are applied in said direction, and a face of abutment against a remaining portion of the bone and/or against surrounding ligaments.

2. Implant according to claim 1, chacterised in that the head (1) is made of pyrocarbon.

3. Implant according to claim 2, **characterised in that** the external surface of the head (1) is polished.

4. Implant according to any one of claims 1 to 3, **characterised in that** the holding element (2) is a stem.

5. Implant according to claim 4, **characterised in that** said direction in which the head (1) is movable in terms of translation is transverse relative to the axis of the stem (2).

6. Implant according to claim 5, **characterised in that** the stem (2) is joined to a nipple (3) inserted in a bore (12) in the head (1), the ability of the head (1) to move in terms of transverse translation relative to the stem (2) being obtained by a play (13) between the nipple (3) and the head (1).

7. Implant according to claim 6, **characterised in that** the respective cross-sections of the nipple (3) and the bore (12) are both circular and the cross-section of the nipple (3) has a diameter smaller than that of the cross-section of the bore (12)

8. Implant according to any one of claims 4 to 7, **characterised in that** the stem (2) is arranged to be held in a hole in said bone simply by elastic deformation.

9. Implant according to any one of claims 1 to 8, **characterised in that** it consists of a partial implant.

10. Implant according to any one of claims 1 to 8, **characterised in that** it consists of an implant of the head of the ulna; and **in that** the articulation face (1b) of the head (1) is provided to articulate with the head of the radius (7).

11. Implant according to claim 10, **characterised in that** it consists of a partial implant of the head of the ulna; and **in that** the abutment face (1a) is provided to abut against a remaining part (6) of the head of the ulna (5).

12. Implant according to any one of the preceding claims, **characterised in that** the articulation face (1 b) of the head (1) is hemispherical; and **in that** the abutment face (1a) is flat.
